# EUROPEAN PATENT APPLICATION

(11) **EP 4 691 490 A1**
(43) Date of publication of application: **11.02.2026**
(21) Application number: 24780780.3
(22) Date of filing: 29.03.2024
(51) Int. Cl.: A61K 45/00, A61K 31/713, A61K 31/7088, A61K 31/7105, A61K 38/48, A61K 39/395, A61K 48/00, A61P 35/00, A61P 35/02

(54) **PHARMACEUTICAL COMPOSITION FOR TREATING AND/OR PREVENTING CANCER**

(30) Priority: 31.03.2023 JP 2023056981
(71) Applicant: Toray Industries, Inc., Tokyo 103-8666 (JP)
(72) Inventor: OKANO Fumiyoshi, Kamakura-shi, Kanagawa 248-8555 (JP); SAITO Takanori, Kamakura-shi, Kanagawa 248-8555 (JP); MINAMIDA Yoshitaka, Kamakura-shi, Kanagawa 248-8555 (JP)
(74) Representative: Mewburn Ellis LLP
(86) International application number: PCT/JP2024/012990
(87) International publication number: WO 2024/204685

(57) **Abstract**

A substance that reduces an expression of a CAPRIN-1 protein on a membrane surface of a cancer cell, specifically a substance that reduces an expression level of the CAPRIN-1 protein on the membrane surface of the cancer cell, or a substance that binds to the CAPRIN-1 protein on the membrane surface of the cancer cell can be used as an active ingredient of a pharmaceutical composition for treating and/or preventing a cancer.

## Description

### Technical Field

The present invention relates to a pharmaceutical composition for treating and/or preventing a cancer, which comprises as an active ingredient a substance that reduces an expression of a CAPRIN-1 protein on a membrane surface of a cancer cell.

### Background Art

In cancer treatment, several treatments using molecular targeting drugs that control the expression or suppress the functions of proteins present in cancer cells have been attempted. Antibody drugs, a type of molecular targeting drug, are used as therapeutic agents with high specificity to targets and few side effects. Examples of the antibody drugs include those that specifically bind to receptors or ligands to block signal transduction, those that improve signal transduction, or those that induce ADCC, CDC, or ADCP after binding to targets. Other molecular targeting drugs include small molecule drugs targeting kinases and small molecule drugs targeting proteasomes. In addition, since technology for conferring resistance to nucleases *in vivo* has improved and efficient uptake into cells has become possible, nucleic acid drugs with even higher target specificity than that of the above-mentioned molecular targeting drugs are beginning to be put into practical use.

Cytoplasmic and proliferation-associated protein 1 (CAPRIN-1) is an intracellular protein that is expressed when dormant normal cells become activated or undergo cell division, and is known to form intracellular stress granules with RNA within cells, so as to be involved in, for example, the transport of mRNA and the control of translation. On the other hand, since the CAPRIN-1 protein is specifically expressed on the cell membrane surface of various cancer cells, it is known that antibodies that specifically bind to the CAPRIN-1 protein expressed on the membrane surface of cancer cells are promising as therapeutic agents for cancers (Patent Literature 1).

### Citation List

### Patent Literature

Patent Literature 1: WO2010/016526

### Summary of Invention

### Technical Problem

An object of the present invention is to provide a novel pharmaceutical product that is useful for treating and/or preventing a cancer.

### Solution to Problem

The present inventors have discovered that tumorigenesis *in vivo* in cancer patients can be suppressed by reducing the expression of a CAPRIN-1 protein on the membrane surface of cancer cells, leading to the idea of using a substance capable of reducing the expression of the CAPRIN-1 protein on the membrane surface of the cancer cells as an active ingredient of a pharmaceutical product for treating and/or preventing a cancer, and thus have completed the present invention.

Specifically, the present invention relates to the following embodiments (1) to (14).
(1) A pharmaceutical composition for treating and/or preventing a cancer, comprising as an active ingredient a substance that reduces an expression of a CAPRIN-1 protein on a membrane surface of a cancer cell.
(2) The pharmaceutical composition according to (1), wherein a cancer patient having the cancer is a cancer patient affected with a cancer in which the CAPRIN-1 protein is expressed on the membrane surface of the cancer cell.
(3) The pharmaceutical composition according to (1) or (2), wherein the substance is a substance that reduces an expression level of the CAPRIN-1 protein on the membrane surface of the cancer cell.
(4) The pharmaceutical composition according to any one of (1) to (3), wherein the substance is at least one substance selected from the group consisting of an AMPK inhibitor, a Wnt agonist, an Akt/PI3K inhibitor, a P53 inhibitor, a Rac1 inhibitor, a ROS generating factor, an ErbB2/HER2 inhibitor, a GSK3β inhibitor, an endocytosis inhibitor and a Wnt/β-catenin inhibitor.
(5) The pharmaceutical composition according to any one of (1) to (3), wherein the substance is a substance that suppresses an expression of a CAPRIN-1 gene.
(6) The pharmaceutical composition according to (5), wherein the substance is a nucleic acid molecule.
(7) The pharmaceutical composition according to (6), wherein the nucleic acid molecule is at least one nucleic acid molecule selected from the group consisting of a small interfering RNA (siRNA), a micro RNA, a small hairpin RNA (shRNA), a guide RNA (gRNA), an antisense nucleic acid, a ribozyme and a nucleic acid aptamer, targeting the CAPRIN-1 gene.
(8) The pharmaceutical composition according to any one of (1) to (3), wherein the substance is an enzyme that degrades the CAPRIN-1 protein on the membrane surface of the cancer cell.
(9) The pharmaceutical composition according to (8), wherein the enzyme is trypsin or chymotrypsin.
(10) The pharmaceutical composition according to (1) or (2), wherein the substance is a substance that binds to the CAPRIN-1 protein on the membrane surface of the cancer cell.
(11) The pharmaceutical composition according to (10), wherein the substance is an antibody that binds to the CAPRIN-1 protein on the membrane surface of the cancer cell or an antigen-binding fragment thereof.
(12) The pharmaceutical composition according to any one of (1) to (11), wherein the cancer is ovarian cancer, bile duct cancer, breast cancer, kidney cancer, pancreatic cancer, colon cancer, melanoma, lung cancer, renal cell carcinoma, head and neck cancer, gastric cancer, biliary tract cancer, brain tumor, prostate cancer, mesothelioma, colorectal cancer, esophageal cancer, gastroesophageal junction cancer, hepatocellular carcinoma, glioblastoma, urothelial carcinoma, bladder cancer, uterine cancer, liver cancer, sarcoma, fibrosarcoma, mast cell tumor, adrenocortical carcinoma, Ewing's tumor, multiple myeloma, testicular cancer, thyroid cancer, basal cell carcinoma, Paget's disease, skin cancer, gastrointestinal stromal tumor (GIST), renal pelvis and ureter cancer, rare cancer, primary central nervous system lymphoma, primary testicular lymphoma, Hodgkin's lymphoma, leukemia or lymphoma.
(13) A method for treating and/or preventing a cancer, comprising administering the pharmaceutical composition according to any one of (1) to (12) to a cancer patient.
(14) The method for treating and/or preventing the cancer according to (13), wherein the cancer patient is a cancer patient affected with a cancer in which a CAPRIN-1 protein is expressed on a membrane surface of a cancer cell.

### Advantageous Effects of Invention

According to the present invention, the expression of the CAPRIN-1 protein on the membrane surface of the cancer cells can be reduced, so that tumorigenesis *in vivo* in the cancer patient can be suppressed.

### Brief Description of Drawing

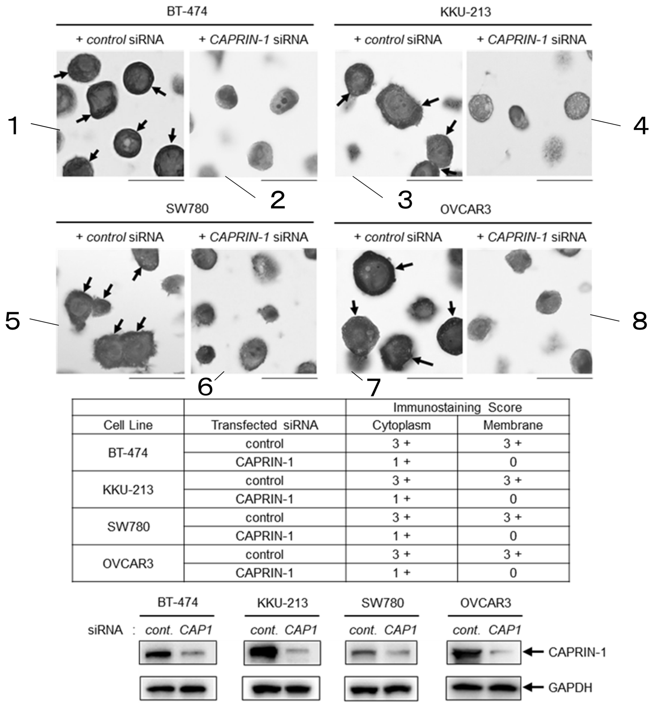

[Figure 1] Figure 1 is a diagram showing changes in the expression of a CAPRIN-1 protein on the cell membrane surface due to the suppressed expression of a CAPRIN-1 gene. The upper panel shows human cancer cell lines BT474, KKU-213, SW780, and OVCAR3 confirmed to express the CAPRIN-1 protein on the cell membrane surface. Reference numbers 1, 3, 5, and 7 depict the expression of the CAPRIN-1 protein on the cell membrane in various cancer cells treated with negative control (+control siRNA) siRNA, and reference numbers 2, 4, 6, and 8 depict the expression of the CAPRIN-1 protein on the cell membrane in various cancer cells treated with siRNA specific to the CAPRIN-1 gene (+CAPRIN-1 siRNA) (the arrows indicate the expression of the CAPRIN-1 protein on the cell membrane surface). A table in the middle panel shows the immunostaining scores of the CAPRIN-1 protein in the cytoplasm (Cytoplasm) and cell membrane (Membrane) of human cancer cells BT474, KKU-213, SW780, and OVCAR3 confirmed to express the CAPRIN-1 protein on the cell membrane surface. The lower panel shows the expression of the CAPRIN-1 protein in various cancer cells when the expression of the CAPRIN-1 gene was suppressed with siRNA in human cancer cells BT474, KKU-213, SW780, and OVCAR3 confirmed to express the CAPRIN-1 protein on the cell membrane surface.

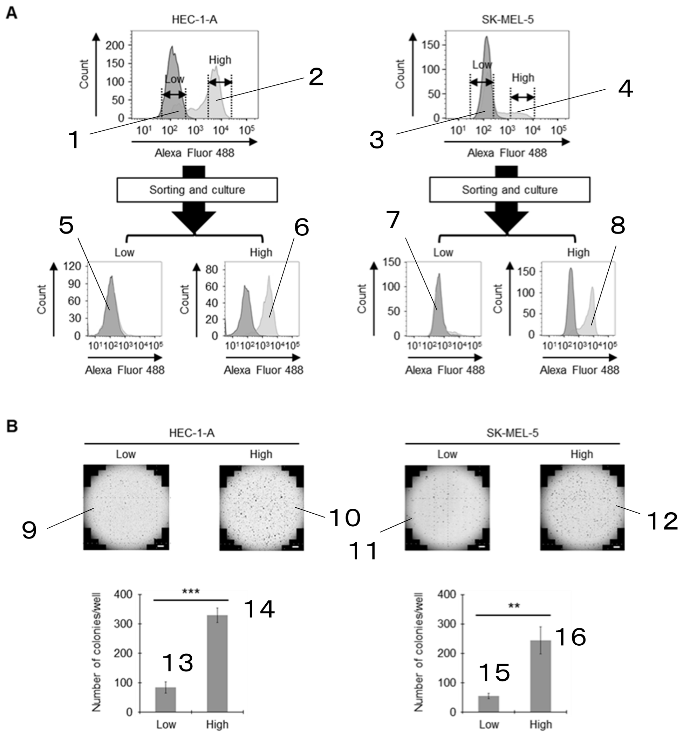

[Figure 2] Figure 2 is a diagram showing the results of sorting cancer cell fractions with different expressions of the CAPRIN-1 protein on the cell membrane surface from human cancer cell lines HEC-1 and SK-MEL-5 confirmed to express the CAPRIN-1 protein on the cell membrane surface, and evaluating the anchorage-independent colony-forming ability of each cancer cell fraction. The upper panel A is a diagram showing the fractionation of cancer cell fractions with different expressions of the CAPRIN-1 protein on the cell membrane surface. Reference numbers 1 and 3 depict cancer cell fractions with high-level expression of the CAPRIN-1 protein on the cell membrane surface, reference numbers 2 and 4 depict cancer cell fractions with low-level expression of the CAPRIN-1 protein on the cell membrane surface, reference numbers 5 and 7 depict cancer cell fractions with low-level expression of the CAPRIN-1 protein on the cell membrane surface after sorting (Low), and reference numbers 6 and 8 depict cancer cell fractions with high-level expression of the CAPRIN-1 protein on the cell membrane surface after sorting (High). The lower panel B is a diagram showing the results of evaluating the anchorage-independent colony-forming ability of each cancer cell fraction. Reference numbers 9 and 11 are images of colony formation in soft agar medium of a cancer cell fraction (Low) sorted from human cancer cells HEC-1-A. Reference numbers 10 and 12 are images of colony formation in soft agar medium of a cancer cell fraction (High) sorted from human cancer cells SK-MEL-5. Reference number 13 depicts the number of colonies formed in soft agar medium by a cancer cell fraction (Low) sorted from human cancer cells HEC-1-A. Reference number 14 depicts the number of colonies formed in soft agar medium by a cancer cell fraction (High) sorted from human cancer cells HEC-1-A. Reference number 15 depicts the number of colonies formed in soft agar medium by a cancer cell fraction (Low) sorted from human cancer cells SK-MEL-5. Reference number 16 depicts the number of colonies formed in soft agar medium by a cancer cell fraction (High) sorted from human cancer cells SK-MEL-5.

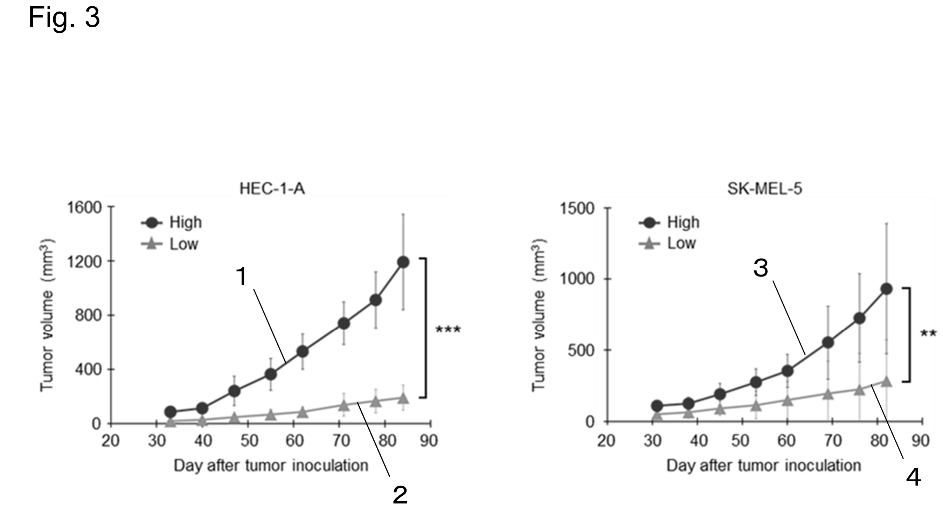

[Figure 3] Figure 3 is a diagram showing differences in tumorigenicity *in vivo* in mice between cancer cell fractions differing in the expression of the CAPRIN-1 protein on the cell membrane surface. Reference number 1 depicts the tumorigenicity *in vivo* in mice of a cancer cell fraction (High) sorted from human cancer cells HEC-1-A. Reference number 2 depicts the tumorigenicity *in vivo* in mice of a cancer cell fraction (Low) sorted from human cancer cells HEC-1-A. Reference number 3 depicts the tumorigenicity *in vivo* in mice of a cancer cell fraction (High) sorted from human cancer cells SK-MEL-5. Reference number 4 depicts the tumorigenicity *in vivo* in mice of a cancer cell fraction (Low) sorted from human cancer cells SK-MEL-5.

### Description of Embodiments

As used herein, the "expression of protein" refers to the generation of a protein from a gene that encodes the protein, and the "suppress expression of protein" refers to completely inhibiting the expression of a protein, or reducing the expression of a protein to a level below that before the treatment, by some kind of treatment.

As used herein, the "suppress expression of gene" refers to completely inhibiting the transcription of mRNA or reducing a transcription level of mRNA compared to that before the treatment, from a gene that encodes a protein by some kind of treatment, thereby completely inhibiting the expression of the protein or reducing the expression of the protein to a level lower than that before the treatment.

As used herein, the "treat (treatment)" refers to a treatment of a cancer based on the antitumor effect of an active ingredient(s) of a pharmaceutical composition. In addition, as used herein, the "prevent (prevention)" refers not only to the prevention of cancer onset, but also to the prevention of cancer metastasis or recurrence.

As used herein, the terms "cancer" and "tumor" refer to malignant neoplasms and are used interchangeably.

The present invention relates to a pharmaceutical composition useful for treating and/or preventing a cancer, which comprises as an active ingredient a substance that reduces an expression of a CAPRIN-1 protein on a membrane surface of a cancer cell.

Examples of the substance that reduces the expression of the CAPRIN-1 protein on the membrane surface of the cancer cells include substances that act inside the cancer cells to directly or indirectly reduce the expression of the CAPRIN-1 protein on the membrane surface of the cancer cells, substances that cleave the polypeptide of the CAPRIN-1 protein on the membrane surface of the cancer cells, as well as substances that bind to the CAPRIN-1 protein on the membrane surface of the cancer cells, specifically substances that bind to the CAPRIN-1 protein on the membrane surface of the cancer cells and directly cover the part or all of the CAPRIN-1 protein on the membrane surface of the cancer cells. The present invention will be described in detail below.

### <Reduction of expression of CAPRIN-1 protein on membrane surface of cancer cells>

Examples of targets of the substance that reduces the expression of the CAPRIN-1 protein on the membrane surface of the cancer cells include CAPRIN-1 genes and proteins. Examples of the CAPRIN-1 genes and proteins include CAPRIN-1 genes and proteins derived from humans, canines, bovines, horses, mice, rats and chickens disclosed in WO2010/016526. In addition, for example, by accessing the GenBank (NCBI, U.S.A.) and utilizing algorithms such as BLAST and FASTA (Karlin and Altschul, Proc. Natl. Acad. Sci. USA, 90:5873-5877, 1993; Altschul et al., Nucleic Acids Res. 25:3389-3402, 1997), the nucleotide sequences and amino acid sequences of the CAPRIN-1 genes and proteins can be obtained.

The expression of the CAPRIN-1 protein can be detected by Western blotting or the like using an anti-CAPRIN-1 antibody. Specific examples of the anti-CAPRIN-1 antibody include anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176, and WO2015/020212.

The reduction in the expression of the CAPRIN-1 protein on the membrane surface of the cancer cells caused by a substance that reduces the expression of the CAPRIN-1 protein on the membrane surface of the cancer cells can be detected using an anti-CAPRIN-1 antibody, as in Western blotting. Specifically, the anti-CAPRIN-1 antibody is reacted with cells whose cell membranes are not subjected to permeabilization, and then a secondary antibody labeled with any detection fluorescent material is reacted therewith, so as to be able to detect the reduction by free cytometry. Alternatively, the reduction can be detected using the anti-CAPRIN-1 antibody directly labeled with any detection fluorescent material. Here, the reduction in the expression of the CAPRIN-1 protein on the membrane surface of the cancer cells refers to a state in which the level of the CAPRIN-1 protein present on the membrane surface of cancer cells (hereinafter referred to as the expression level) is reduced, or a state in which part or all of the CAPRIN-1 protein present on the membrane surface of the cancer cells is covered by a substance that binds to the CAPRIN-1 protein on the membrane surface of the cancer cells. Although described in detail below, examples of substances that reduce the expression level of the CAPRIN-1 protein present on the membrane surface of the cancer cells include low molecular weight compounds, substances that suppress the expression of a CAPRIN-1 gene, and enzymes that degrade the CAPRIN-1 protein on the membrane surface of the cancer cells. Examples of substances that cover part or all of the CAPRIN-1 protein on the membrane surface of the cancer cells include substances that bind to the CAPRIN-1 protein on the membrane surface of the cancer cells. When evaluation is performed by flow cytometry using an anti-CAPRIN-1 antibody reactive with the CAPRIN-1 protein on the cell membrane surface, the evaluation can be performed as follows. When the expression of the CAPRIN-1 protein on the cell membrane surface of untreated cells is expressed as mean fluorescence intensity and the resultant is taken as a reference value of 100%, a value of the above mean fluorescence intensity in cells treated with a substance that reduces the expression of the CAPRIN-1 protein on the membrane surface of the cancer cells is obtained by, for example, using the following formula (suppression degree (%) of the expression of the CAPRIN-1 protein on the cell membrane surface = mean fluorescence intensity of cells reacted with a factor / mean fluorescence intensity of untreated cells × 100). In the present invention, successful suppression may be represented by a significant difference, preferably that is the suppression degree (%) obtained in the above evaluation of, 95% or less, 90% or less, 80% or less, 70% or less, 60% or less, 50% or less, or 40% or less.

### <Substances that reduce expression of CAPRIN-1 protein on the membrane surface of cancer cells>

In the present invention, examples of the substances that reduce the expression of the CAPRIN-1 protein on the membrane surface of the cancer cells include low molecular weight compounds, substances that suppress the expression of the CAPRIN-1 gene, and enzymes that degrade the CAPRIN-1 protein on the membrane surface of the cancer cells.

The substance that reduces the expression of the CAPRIN-1 protein on the membrane surface of the cancer cells used in the present invention is not particularly limited as long as it can reduce the expression of the CAPRIN-1 protein on the membrane surface of the cancer cells. Specific examples of the substances that reduce the expression level of the CAPRIN-1 protein on the membrane surface of the cancer cells include low molecular weight compounds having a function of reducing the expression level of the CAPRIN-1 protein on the membrane surface of the cancer cells, substances that suppress the expression of the CAPRIN-1 gene (substances that suppress the transcription of mRNA from the CAPRIN-1 gene, substances that inhibit the translation of the CAPRIN-1 protein from mRNA transcribed from the CAPRIN-1 gene, substances that degrade mRNA transcribed from the CAPRIN-1 gene, or substances that degrade the CAPRIN-1 protein translated from mRNA transcribed from the CAPRIN-1 gene), or hydrolases that degrade the CAPRIN-1 protein on the membrane surface of the cancer cells.

Specific examples of the low molecular weight compounds having the function of reducing the expression level of the CAPRIN-1 protein on the membrane surface of the cancer cells include, but are not limited to, AMPK inhibitors, IFG-1R inhibitors, Wnt agonists, Akt/PI3K inhibitors, P53 inhibitors, Rac1 inhibitors, ROS generating factors, ErbB2/HER2 inhibitors, GSK3β inhibitors, IGF-1R inhibitors, endocytosis inhibitors, and Wnt/β-catenin inhibitors, and preferred specific examples thereof include, but are not limited to, Dorsomorphin as the AMPK inhibitor, Wnt agonist 1 as the Wnt agonist, LY294002 as the Akt/PI3K inhibitor, Pifithrin-α as the P53 inhibitor, a Rac1 inhibitor (CAS#1177865-17-6) as the Rac1 inhibitor, Pyocyanin as the ROS generating factor, Mubritinib as the ErbB2/HER2 inhibitor, GSK-3β inhibitor VIII as the GSK3β inhibitor, Dynasore as the endocytosis inhibitor, and Wnt-C59 as the Wnt/β-catenin inhibitor.

A preferred aspect of the substances that suppress the expression of the CAPRIN-1 gene is a nucleic acid molecule. The nucleic acid molecule that suppresses the expression of the CAPRIN-1 gene is not particularly limited as long as it is a nucleic acid molecule that can bind to the full length or a partial sequence of the CAPRIN-1 gene to suppress the expression of the CAPRIN-1 gene, and examples of such a nucleic acid molecule can include a nucleic acid molecule that suppresses the transcription of the CAPRIN-1 gene, a nucleic acid molecule that disrupts the CAPRIN-1 gene, and a nucleic acid molecule that suppresses the translation of the CAPRIN-1 gene. Specifically, examples thereof can include siRNA, microRNA, or shRNA targeting the full length or the partial sequence of the CAPRIN-1 gene, the full length or the partial sequence itself of the CAPRIN-1 gene, or guide RNA targeting protospacer adjacent motifs (NGC sequence, NNGRR sequence, NNNNRYAC sequence, TTN sequence, TTTR sequence, 3'A/U/C sequence), an antisense nucleic acid targeting the full length or the partial sequence of the CAPRIN-1 gene, a ribozyme targeting the full length or the partial sequence of the CAPRIN-1 gene, and a nucleic acid aptamer (DNA or RNA) that specifically binds to the full length or the partial sequence of the CAPRIN-1 gene. Examples of the above CAPRIN-1 gene include not only a structural gene of the CAPRIN-1 protein, but also transcriptional regulatory regions of the structural gene, such as 5'-UTR and 3'-UTR, a promoter region, and an enhancer region. Specific examples of nucleic acid molecules that suppress the expression of the CAPRIN-1 gene may be substances that suppress the transcription of mRNA from the CAPRIN-1 gene, substances that inhibit the translation of the CAPRIN-1 protein from mRNA transcribed from the CAPRIN-1 gene, substances that degrade mRNA transcribed from the CAPRIN-1 gene, or substances that degrade the CAPRIN-1 protein translated from mRNA transcribed from the CAPRIN-1 gene. In addition, in the case of a nucleic acid molecule complementary to the CAPRIN-1 gene, the nucleic acid molecule is preferably completely complementary to the nucleotide sequence of the CAPRIN-1 gene, but under stringent conditions, it is sufficient that it hybridizes, and the homology of the complementary sequence is preferably 90% or more, and more preferably 95% or more.

The nucleic acid molecule can be designed by methods well-known to a person skilled in the art based on information on a sequence of the CAPRIN-1 gene, using indicators such as a G (guanine) C (cytosine) content, a sequence specificity, an off-target effect, and the like. For example, in the case of siRNA, an siRNA sequence can be designed from the nucleotide sequence of the CAPRIN-1 gene using a database such as siDirect (http://sidirect2.rnai.jp), and chemically synthesized by methods well-known to a person skilled in the art.

In one preferred aspect, the nucleic acid molecule is used in combination with a pharmaceutically acceptable carrier. The carrier is not particularly limited, but is preferably a carrier in a form that enhances accumulation to cancer tissues expressing the CAPRIN-1 gene, a form that enhances intracellular permeability, or a form that enhances the stability of the nucleic acid molecule. For example, cationic liposomes can be used (refer to Yano J et al., Clinical Cancer research, 10(22), 7721, 2004). Examples of the other carriers include particles, lipid particles, or lipid nanoparticles, containing cationic lipids, neutral lipids cholesterol lipids or PEG-modified lipids. Alternatively, examples thereof include amphiphilic polymers described in WO2023/042872, WO2023/032892, and WO22023/032891, and polymers described in WO2016/066586, WO2010/005721, WO2016/187234, WO2016/123480, WO2006/107903, WO2010/005740, WO2017/003668, WO2007/056153, and WO2002/080982.

Further, chemical modifications may be performed to prevent *in vivo* degradation of nucleic acid molecules (refer to Ross J. et al., Nature, 432 (7014), 155, 2004).

Further, a vector that expresses the nucleic acid molecule can be used. Specific examples thereof include viral vectors (e.g., adenovirus vectors) and non-viral vectors (e.g., plasmid DNA).

Specific examples of the enzymes that degrade the CAPRIN-1 protein on the membrane surface of the cancer cells include trypsin and chymotrypsin.

Furthermore, examples of the substances that reduce the expression of the CAPRIN-1 protein on the membrane surface of the cancer cells also include substances that bind to the CAPRIN-1 protein on the membrane surface of the cancer cells to cover part or all of the CAPRIN-1 protein, or substances that bind to the CAPRIN-1 protein on the membrane surface of the cancer cells to internalize the CAPRIN-1 protein into the cells. These substances may be substances that can directly damage cancer cells expressing the CAPRIN-1 protein on the cell membrane surface or that induce a cytotoxic activity against the cancer cells, but the effects of the present invention can be obtained even with substances that do not directly damage the cancer cells or that do not induce the cytotoxic activity against the cancer cells. A specific preferred example thereof is an antibody that binds to the CAPRIN-1 protein on the membrane surface of the cancer cells or an antigen-binding fragment thereof (hereinafter also referred to as a CAPRIN-1 expression-inhibiting antibody). The CAPRIN-1 expression-inhibiting antibody may be the one capable of directly damaging cancer cells expressing the CAPRIN-1 protein on the cell membrane surface, or the one capable of inducing cytotoxic activity against the cancer cells, or may be the one that does not directly damage the cancer cells, or the one that does not induce the cytotoxic activity against the cancer cells.

The antibody type of the CAPRIN-1 expression-inhibiting antibody is not particularly limited, and may be any class and isotype, such as IgA, IgD, IgE, IgG1, IgG2a, IgG2b, IgG3, and IgM and the like. Examples of the antigen-binding fragment include Fab, Fv, F(ab')₂, and Fab'.

The CAPRIN-1 expression-inhibiting antibody may be a monoclonal antibody or a polyclonal antibody.

The polyclonal antibody can be obtained, for example, by immunizing mice, human antibody-producing mice, rats, rabbits, chickens, etc., with a natural CAPRIN-1 protein or a fusion protein with GST or the like, or a partial peptide thereof, and then obtaining serum, and purifying the obtained serum by ammonium sulfate precipitation, protein A, protein G, a DEAE ion exchange column, an affinity column with the CAPRIN-1 protein or a partial peptide, etc., bound thereto.

The method for preparing the CAPRIN-1 protein used in the above immunization can be obtained by referring to WO2014/012479, and cells or the like expressing the CAPRIN-1 protein can also be used.

The monoclonal antibody can be obtained, for example, by immunizing a mouse through administration of SK-BR-3 breast cancer cells expressing the CAPRIN-1 protein or the full-length CAPRIN-1 protein or a fragment thereof, or the like, fusing spleen cells isolated from the mouse with myeloma cells, and selecting a clone that produces the monoclonal antibody from the resulting fused cells (hybridoma). The antibody produced by the selected hybridoma can be obtained by a method similar to the above purification method for polyclonal antibodies.

Examples of the antibodies to be used in the present invention include human antibodies, humanized antibodies, chimeric antibodies, and non-human animal antibodies.

Human antibodies can be obtained by sensitizing human lymphocytes infected with EB virus with a protein, protein-expressing cells or a lysate thereof, fusing the sensitized lymphocytes with myeloma cells such as human-derived U266 cells, and then obtaining antibodies that are immunologically reactive with the full-length CAPRIN-1 protein or a fragment thereof from the resulting fused cells.

A humanized antibody is a modified antibody also referred to as a reshaped human antibody. The humanized antibody is constructed by transplanting a complementarity determining region of an antibody derived from an immunized animal into a complementarity determining region of a human antibody. As a common technique for that, a gene recombination technique is also well-known technology. Specifically, for example, a DNA sequence designed to ligate a complementarity determining region of a mouse antibody or a rabbit antibody with the framework region of a human antibody is synthesized by PCR with the use of several oligonucleotides prepared to have overlapping portions at the ends. The thus obtained DNA is ligated to DNA encoding a constant region of a human antibody, the resultant is incorporated into an expression vector, and then the expression vector is introduced into a host for production (refer to EP239400 and WO96/02576). The framework region of the human antibody to be ligated via the complementarity determining region is selected, so that the complementarity determining region forms a good antigen binding site. If necessary, amino acids in the framework region in a variable region of the antibody may be substituted so that the complementarity determining regions of the reshaped human antibody form an appropriate antigen-binding site (Sato K. et al., Cancer Research 1993, 53: 851-856). Alternatively, they may be substituted with framework regions derived from various human antibodies (refer to WO99/51743).

The antibodies are heteromeric glycoproteins that usually contain at least two heavy chains and two light chains. The antibody is composed of two identical light chains and two identical heavy chains. The heavy chain has a heavy chain variable region at one end followed by several constant regions. The light chain has a light chain variable region at one end followed by several constant regions. The variable regions have specific variable regions called complementarity determining regions (CDRs) that confer binding specificity to the antibody. Relatively conserved portions of the variable regions are called framework regions (FRs). The variable regions in complete heavy chains and light chains each contain four FRs linked by three CDRs (CDR1-CDR3).

The sequences of the constant regions and variable regions in human-derived heavy chains and light chains are available, for example, from the NCBI (USA: GenBank, UniGene, etc.). For example, reference can be made to the sequences of the human IgG1 heavy chain constant region under registration number J00228, the human IgG2 heavy chain constant region under registration number J00230, the human light chain κ constant region under registration numbers V00557, X64135, X64133, etc., and the human light chain λ constant region under registration numbers X64132, X64134, etc.

The chimeric antibodies are antibodies prepared by combining sequences derived from different animals, such as an antibody composed of a heavy chain variable region and a light chain variable region of a mouse antibody and a constant region of a heavy chain constant region variable region and a light chain constant region variable region of a human antibody, and the like. The chimeric antibodies can be prepared using known methods, for example, by ligating DNA encoding a V region of an antibody with DNA encoding a C region of a human antibody, incorporating this into an expression vector, and then introducing the expression vector into a host for production.

The non-human animal antibodies can be obtained by immunizing animals with sensitizing antigens according to known methods. A general method involves injecting a sensitizing antigen into animals such as mice intraperitoneally, intradermally, or subcutaneously. When injecting the sensitizing antigen, it is mixed in an appropriate amount with various adjuvants including CFA (Freund's complete adjuvant) and then administered to an animal multiple times. After immunizing the animal and confirming that the serum contains an anti-CAPRIN-1 antibody, the serum is obtained and then purified as described above using ammonium sulfate precipitation, protein A, protein G, a DEAE ion exchange column, or an affinity column with the CAPRIN-1 protein or partial peptides thereof bound thereto, so that a non-human animal antibody can be obtained. In addition, when obtaining a monoclonal antibody from a non-human animal, immune cells can be collected from the immunized animal and then subjected to cell fusion with myeloma cells, so that the monoclonal antibody can be obtained. The cell fusion of the immune cells with the myeloma cells can be carried out according to known methods (refer to Kohler, G. and Milstein, C. Methods Enzymol. (1981) 73, 3-46).

The CAPRIN-1 expression-inhibiting antibody can also be obtained as a recombinant type antibody produced by cloning an antibody gene from a hybridoma, incorporating it into a suitable vector, and then introducing the resultant into a host for production using recombinant gene technology (refer to Carl, A.K. Borrebaeck, James, W. Larrick, THERAPEUTIC MONOCLONAL ANTIBODIES, Published in the United Kingdom by MACMILLAN PUBLISHERS LTD, 1990).

The CAPRIN-1 expression-inhibiting antibody having higher binding affinity for the CAPRIN-1 protein on the membrane surface of the cancer cells is expected to have a stronger antitumor effect. The binding constant (affinity constant) Ka (kon/koff) is preferably at least 10⁷ M⁻¹ , at least 10⁸ M⁻¹ , at least 5 × 10⁸ M⁻¹ , at least 10⁹ M⁻¹ , at least 5 × 10⁹ M⁻¹ , at least 10¹⁰ M⁻¹, at least 5 × 10¹⁰ M⁻¹, at least 10¹¹ M⁻¹, at least 5 × 10¹¹ M⁻¹, at least 10¹² M⁻¹, or desirably at least 10¹³ M⁻¹.

The ability of the CAPRIN-1 expression-inhibiting antibody to bind to the CAPRIN-1 protein on the membrane surface of the cancer cells can be specified using binding assays, such as ELISA, Western blotting, immunofluorescence, flow cytometry and the like.

The CAPRIN-1 expression-inhibiting antibody may be chemically modified. Examples of such modified antibodies can include antibodies bound to various molecules such as polyethylene glycol (PEG) and the like. There are no limitations on the substance to be bound in the modified antibody of the present invention. Such a modified antibody can be obtained by chemically modifying the obtained antibody. These methods have already been established in this field.

The CAPRIN-1 expression-inhibiting antibody can improve the ability of an anti-CAPRIN-1 antibody to bind to effector cells through substitution of one, two or several amino acids of heavy chain constant regions of the antibody, or through the removal of fucose attached to N-acetylglucosamine in the N-glycoside-linked glycan that is bound to the heavy chain constant regions. The above may be an amino acid substitution alone, or may be a composition formed with an antibody containing fucose attached thereto.

Antibodies in which one, two or several amino acids in the heavy chain constant regions have been substituted can be prepared by referring to, for example, WO2004/063351, WO2011/120135, U.S. Patent No. 8,388,955, WO2011/005481, U.S. Patent No. 6,737,056, and WO2005/063351.

An antibody from which fucose attached to N-acetylglucosamine in the N-glycoside-linked glycan in the heavy chain constant regions has been removed, or a cell producing the antibody, can be prepared by referring to U.S. Patent No. 6,602,684, European Patent No. 1,914,244, and U.S. Patent No. 7,579,170. A composition of an antibody from which fucose attached to N-acetylglucosamine in the N-glycoside-linked glycan that is bound to the heavy chain constant regions has been removed and an antibody to which fucose is attached, or a cell producing the same, can be prepared by referring to, for example, U.S. Patent No. 8,642,292.

Specific examples of the CAPRIN-1 expression-inhibiting antibody include antibodies that can be prepared referring to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176, and WO2015/020212.

### <Pharmaceutical Composition>

An object of the pharmaceutical composition of the present invention, which comprises as an active ingredient a substance that reduces the expression of the CAPRIN-1 protein on the membrane surface of the cancer cells, is to treat and/or prevent a cancer. The CAPRIN-1 protein is prominently expressed on the cell membrane surface of various cancer cells. The substance that reduces the expression of the CAPRIN-1 protein on the membrane surface of the cancer cells, which is an active ingredient of the pharmaceutical composition of the present invention, can reduce the tumorigenicity of the cancer cells by reducing the expression of the CAPRIN-1 protein on the cell membrane surface of various cancer cells. The antitumor activity of the substance that reduces the expression of the CAPRIN-1 protein on the membrane surface of the cancer cells can be evaluated by examining the suppression of the anchorage-independent colony-forming ability of the cancer cells and the proportion of dead cells *in vitro,* and by examining the suppression of tumorigenesis or tumor growth *in vivo* in mice, as described below.

Cancers targeted by the pharmaceutical composition of the present invention are not particularly limited as long as the CAPRIN-1 protein is expressed on the cell membrane surface, but are preferably, ovarian cancer, bile duct cancer, breast cancer, kidney cancer, pancreatic cancer, colon cancer, melanoma (including postoperative melanoma), lung cancer (including non-small cell lung cancer and small cell lung cancer), renal cell carcinoma, head and neck cancer, gastric cancer, biliary tract cancer, brain tumor, prostate cancer, mesothelioma (including malignant pleural mesothelioma), colorectal cancer (e.g., colorectal cancer with MSI-high), esophageal cancer, gastroesophageal junction cancer, hepatocellular carcinoma, glioblastoma, urothelial carcinoma, bladder cancer, uterine cancer (including cervical cancer and uterine body cancer), liver cancer, sarcoma (including osteosarcoma, fibrosarcoma, chondrosarcoma, rhabdomyosarcoma, leiomyosarcoma, liposarcoma, angiosarcoma, cutaneous angiosarcoma, soft tissue sarcoma, head and neck sarcoma, cardiac sarcoma, uterine sarcoma, uterine carcinosarcoma, endometrial stromal sarcoma, uterine leiomyosarcoma, undifferentiated endometrial sarcoma, retroperitoneal sarcoma, childhood sarcoma, alveolar soft part sarcoma, clear cell sarcoma, dermatofibrosarcoma protuberans, and epithelioid sarcoma), mast cell tumor, adrenal cortical carcinoma, Ewing's tumor, multiple myeloma, testicular cancer, thyroid cancer, basal cell carcinoma, Paget's disease, skin cancer, gastrointestinal stromal tumor (GIST), renal pelvis and ureter cancer, rare cancers (eye tumor, orbital sarcoma, eyelid tumor, intracarcinoma lymphoma, adnexal lymphoma, optic nerve tumor, choroidal malignant melanoma, retinoblastoma, lacrimal gland cancer, adenoid cystic carcinoma, auditory cancer, oral cancer, male breast cancer, special breast cancer, malignant mammary phyllodes tumor, malignant pericardial mesothelioma, malignant pleural mesothelioma, thymoma, thymic carcinoma, SMARC4-deficient tumor of the chest, cancer derived from neuroendocrine cells (including NEC, NET, pulmonary neuroendocrine tumor, and gastrointestinal endocrine tumor), malignant peritoneal mesothelioma, anal cancer, anal canal squamous cell carcinoma, small intestine cancer, duodenal cancer, jejunal cancer, ileal cancer, pancreatic neuroendocrine tumor, pheochromocytoma, paraganglioma, malignant tunica vaginalis mesothelioma, vaginal cancer, urachal cancer, peritoneal cancer, sweat gland cancer, sebaceous gland cancer, skin adnexal cancer, Menkel cell carcinoma, squamous cell carcinoma, sarcoma of the trunk, desmoid tumor, germ cell tumor, internal ear canal cancer, external ear canal cancer, gingival cancer (including mandibular gingival cancer), gastrinoma, diffusive desmoid tumor, tongue cancer, urachal cancer, malignant uveal melanoma), primary central nervous system lymphoma, primary testicular lymphoma, Hodgkin's lymphoma, leukemia, or lymphoma. The above cancer may be primary cancer, cancer of unknown primary origin, metastatic cancer, metastasized or recurrent cancer, postoperative cancer, or unresectable cancer. In addition, melanoma is often used synonymously with aggressive melanoma or malignant melanoma.

A cancer patient (subject) to whom the pharmaceutical composition of the present invention is administered may be a cancer patient confirmed to have a cancer or a patient suspected to have a cancer, but is preferably a cancer patient affected with a cancer in which the CAPRIN-1 protein is expressed on the membrane surface of the cancer cells. Here, the "cancer patient affected with a cancer in which the CAPRIN-1 protein is expressed on the membrane surface of the cancer cells" as mentioned herein includes not only cancer patients confirmed to have the cancer at the time of administration of the pharmaceutical composition of the present invention, but also cancer patients confirmed to have had the cancer in the past, and patients to whom the pharmaceutical composition of the present invention is administered to prevent the recurrence of the cancer. Whether or not a patient is affected with a cancer in which the CAPRIN-1 protein is expressed on the membrane surface of the cancer cells can be confirmed, for example, by subjecting the cancer cells/cancer tissues isolated from the cancer patient to the detection of the CAPRIN-1 protein expressed on the membrane surface of the cancer cells, for example, by the method disclosed in WO2010/016527. In terms of biological classification of cancer patients (subjects), mammals such as humans, pet animals, livestock, and sports animals are preferred, with humans being more preferred.

The pharmaceutical composition of the present invention can be formulated by methods known to a person skilled in the art. The pharmaceutical product of the present invention can be used parenterally, for example, in the form of an injection of a sterile solution or suspension in water or the other pharmaceutically acceptable liquids. In the pharmaceutical product of the present invention, the active ingredient (the ingredient that suppresses the expression of the CAPRIN-1 protein on the membrane surface of the cancer cells) for each formulation or pharmaceutical composition may be appropriately combined with, for example, a pharmacologically acceptable carrier, medium, or additive, specifically, a sterilized water, a physiological saline, an isotonic solution, a buffer (buffer solution, etc.), a vegetable oil, an oily liquid, an antioxidant, a solubilizer, an emulsifier, a suspending agent, a surfactant, a stabilizer, a fragrance, an excipient, a binder, etc., and may be preferably formulated by mixing with them in a unit dose form required for generally accepted pharmaceutical practice. The amount of an active ingredient in these formulations is determined so that an appropriate dose within the indicated range can be obtained.

Sterile compositions for injection can be prescribed according to usual pharmaceutical practice using a vehicle such as distilled water for injection. Aqueous solutions for injection may be formulated with, for example, physiological saline, isotonic solutions containing D-sorbitol, D-mannose, D-mannitol, or other auxiliary agents, alcohols (a specific example is ethanol), polyalcohols (specific examples thereof include propylene glycol and polyethylene glycol), nonionic surfactants (specific examples thereof include polysorbate 80 (TM) and HCO-60), solubilizers such as benzyl benzoate and benzyl alcohol, oily liquids (specific examples thereof include sesame oil and soybean oil), buffers (specific examples thereof include phosphate buffer solution and sodium acetate buffer solution), soothing agents (specific examples thereof include procaine hydrochloride), stabilizers (specific examples thereof include benzyl alcohol and phenol), and antioxidants. The prepared injection solution is usually filled into an appropriate ampule.

Administration may be oral or parenteral, preferably parenteral, and specific examples of the form of administration include injection form, nasal administration form, pulmonary administration form, and transdermal administration form. Examples of the injection form include intravenous injection, intramuscular injection, intraperitoneal injection, subcutaneous injection, and intratumoral injection, which can be administered systemically or locally. Examples of the transdermal administration form include those referred to as liniments or topical agents. Examples of the topical agents include solids, liquids, sprays, ointments, creams, and gels.

Furthermore, an appropriate administration method can be selected depending on the age, body weight, sex, symptoms, etc., of a patient. The dosage of the pharmaceutical composition containing at least one of the active ingredients of the pharmaceutical product of the present invention can be selected in terms of the amount of each active ingredient, for example, from the range of 0.0001 mg to 1,000 mg per kg body weight per administration. Alternatively, the dosage of each active ingredient can be selected from the range of, for example, 0.001 to 100,000 mg/body per patient, or, for example, 1 mg to 30 mg per kg body weight of the patient, but is not necessarily limited to these numerical values. The dosage and administration method vary depending on the patient's body weight, age, sex, symptoms, etc., but can be appropriately selected by a person skilled in the art.

### <Methods for treating and/or preventing cancer>

By administering the pharmaceutical composition of the present invention to the cancer patient (subject) based on the above-mentioned method, the cancer of the cancer patient can be treated and/or prevented. The cancer patient (subject) to be subjected to treatment and/or prevention may be any cancer patient confirmed to have a cancer or any patient suspected to have a cancer, but is preferably a cancer patient affected with a cancer in which the CAPRIN-1 protein is expressed on the membrane surface of the cancer cells. In addition, the pharmaceutical composition of the present invention and another antitumor agent (such as a known antitumor agent) may also be administered to the cancer patient (subject) simultaneously or separately.

### Examples

Hereinafter, the present invention will be described more specifically with reference to Examples. However, the scope of the present invention is not intended to be limited by these Examples.

### (Example 1) Expression of CAPRIN-1 gene in cancer cells

The expression of a CAPRIN-1 gene in various cell lines was examined by RT-PCR (Reverse Transcription-PCR). Reverse transcription reaction was performed as follows. Specifically, total RNA was extracted from 50 mg to 100 mg of each tissue and 5 to 10 × 10⁶ cells of each cell line using a TRIZOL reagent (manufactured by Invitrogen) according to the attached protocol. Using this total RNA, cDNA was synthesized using Superscript First-Strand Synthesis System for RT-PCR (manufactured by Invitrogen) according to the attached protocol. PCR was carried out using the obtained gene-specific primers (sense: 5'-AAGGTTTGAATGGAGTGC-3' (SEQ ID NO: 1) and antisense: 5'-TGCTCCTTTTCACCACTG-3' (SEQ ID NO: 2)) as follows. That is, each reagent and the attached buffer were added in such a manner that a total volume was 25 µl and the resultant contained 0.25 µl of the sample prepared by reverse transcription reaction, 2 µM each of the above primers, 0.2 mM of each dNTP, and 0.65 U of ExTaq polymerase (manufactured by Takara Shuzo Co., Ltd.), and then a cycle of 94°C/30 seconds, 60°C/30 seconds, and 72°C/30 seconds was repeated 30 times using a Thermal Cycler (manufactured by Bio-Rad Laboratories, Inc.). The gene-specific primers amplify a region from positions 206 to 632 from the 5'-end in the nucleotide sequence of the canine CAPRIN-1 gene and a region from positions 698 to 1124 from the 5'-end in the nucleotide sequence of the human CAPRIN-1 gene. For comparison, GAPDH-specific primers (sense: 5'-GGGCTGCTTTTAACTCTG-3' (SEQ ID NO: 3) and antisense: 5'-TGCTCCTTTTCACCACTG-3' (SEQ ID NO: 4)) were also used simultaneously.

As a result, in the cancer cells, the expression of the CAPRIN-1 gene was detected in many types of cancer cell lines. Specifically, the expression of the CAPRIN-1 gene was confirmed in human breast cancer cells BT-474 (HTB-20), MCF7 (HTB-22), T-47D (HTB-133), SK-BR-3 (HTB-30), HCC70 (CRL-2315), BT-20 (HTB-19), MDA-MB-231 (CRM-HTB-26), MDA-MB-453 (HTB-131), and MDA-MB-468 (HTB-132), human bladder cancer cells SW780 (CRL-2169), T24 (HTB-4), HT-1197 (CRL-1473), RT4 (HTB-2), TCCSUP (HTB-5) and HT-1376 (CRL-1472), human pancreatic cancer cells Panc10.05 (CRL-2547), PANC1 (CRL-1469), and SU. 86.86 (CRL-1837), human colon cancer cells HT-29 (HTB-38), HCT-116 (CCL-247), COLO 205 (CCL-222), DLD-1 (CCL-221), and LoVo (CCl-229), human gastric cancer cells NCI-N87 (CRL-5822), human prostate cancer 22Rv1 (CRL-2505), human lung cancer cells A549 (CCL-185), NCI-H2228 (CRL-5935), human kidney cancer cells ACHN (CRL-1611), Caki-1 (HTB-46), and Caki-2 (HTB-47), human liver cancer cells Hep3B (HB-8064), human head and neck cancer cells FaDu (HTB-43), human ovarian cancer cells TOV-21G (CRL-11730), SKOV3 (HTB-77), OVCAR3 (HTB-161), and OV90 (CRL-11732), human uterine cancer cells HEC-1-A (HTB-112), human prostate cancer cells PC-3 (JCRB9110), and DU-145 (HTB-81), human melanoma Malme-3M (HTB-64), SK-MEL-5 (HTB-70), A-375 (CRL-1619), G-361 (CRL-1424), and SK-MEL-28 (HTB-72), human brain tumor cells U-87MG (HTB-14), human lymphoma Ramos (CRL-1596), human fibrosarcoma cells HT-1080 (CCL-121), mouse breast cancer cells 4T1 (CRL-2539), and mouse renal cancer cells Renca (CRL-2947), all of which are available from the American Type Culture Collection (ATCC), human gallbladder cancer cells KKU-213 (JCRB1557), human cholangiocarcinoma HuCCA-1 (JCRB1657), HuCCT-1 (JCRB0425), and KKU-100 (JCRB1568), human breast cancer cells MRK-nu-1 (JCRB0628), human liver cancer cells HepG2 (JCRB1054), human ovarian cancer cells MCAS (JCRB0240), and OVISE (JCRB1043), human pancreatic cancer cells MIA PaCa-2 (JCRB0070), human gastric cancer cells IM95 (JCRB1075.0), MKN1 (JCRB0252), MKN74 (JCRB0255), NUGC-2 (JCRB0921), NUGC-3 (JCRB0822), and NUGC-4 (JCRB0834), all of which are available from the Japanese Collection of Research Bioresources (JCRB), human gallbladder cancer cells TGBC14TKB (RCB1186) available from the RIKEN Cell Bank, as well as human esophageal cancer cells OE33 and human leukemia cells OCI-AML5 (ACC247). These results confirmed that the CAPRIN-1 gene was expressed in many cancer cells.

### (Example 2) Expression of CAPRIN-1 protein in cancer cells

The expression of the CAPRIN-1 protein in the cancer cells used in Example 1 was confirmed by Western blotting. Cells were dispersed in a protein extraction solution, a RIPA buffer solution (Nacalai Tesque, 16488-34) containing a protease inhibitor (Nacalai Tesque, 25955-24) to prepare a cell lysate, the cell lysate was then mixed with a sample buffer solution containing DTT to separate fractions with different molecular weights in acrylamide gel, and then each transferred to a PVDF membrane. Anti-GPIP137 antibodies (Invitrogen, 703658), which were anti-CAPRIN-1 antibodies, were reacted with the PVDF membrane subjected to transfer by a predetermined method, and then the membrane was blocked with a TBS-T solution containing 5% skim milk, followed by reaction with an anti-rabbit IgG antibody labeled with HRP. After reacting with the secondary antibody, Western Lightning Plus-ECL was reacted for detection with an imaging system FUSION SOLO. 6S. EDGE (Vilber Lourmat). As a result, it was confirmed that the CAPRIN-1 protein was expressed in the cancer cells described in Example 1.

### (Example 3) Expression of CAPRIN-1 Protein on the membrane surface of cancer cells

The expression of the CAPRIN-1 protein on the cell membrane surface of the cancer cells for which the expression of the CAPRIN-1 gene was confirmed in Example 1 was confirmed by flow cytometry. 2 × 10⁵ cancer cells suspended in Dulbecco's PBS(-) containing 1% fetal bovine serum (hereinafter, 1% FCS-PBS(-)) were reacted with an antibody against CAPRIN-1 at a concentration of 1 to 10 µg/mL, and then allowed to react on ice (or at 4°C) for 1 hour.

As the anti-CAPRIN-1 antibodies, anti-CAPRIN-1 antibodies described in WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212 were used.

As a negative control, cells that had been reacted with the similar concentration of an isotype antibody that is not reactive with the CAPRIN-1 protein were used.

After the antibody reaction, cells were washed with 1% FCS-PBS (-) and then reacted with a fluorescently labeled anti-IgG secondary antibodies (FITC Goat anti-rabbit IgG (BD Biosciences, 554020), Alexa Fluor 488 anti-rabbit IgG (H+L) (Invitrogen, A21206), Alexa Fluor 647 anti-rabbit IgG (BioLegend, 406414), Alexa Fluor 488 F(ab')2 anti-mouse IgG (H+L) (Invitrogen, A11017) or Alexa Fluor 488 anti-human IgG (H+L) (Invitrogen, A11013) at a concentration of 1 to 10 µg/mL for reaction with an antibody against CAPRIN-1, followed by 1 hour of reaction on ice (or at 4°C). After reaction with the fluorescently labeled secondary antibodies, cells were washed with 1% FCS-PBS(-) and then reacted with a PI (propidium iodide) solution (DOJINDO, 341-07881) or Fixable Viability Stain 450 (BD Horizon) prepared at a concentration of 0.1 µg/mL for 15 minutes. Cells were then washed with 1% FCS-PBS(-) and then the scattered light intensity and fluorescence intensity were measured using a flow cytometer according to a standard method. As a result, the cells reacted with the anti-CAPRIN-1 antibody exhibited stronger fluorescence intensity than the negative control. This confirmed the expression of the CAPRIN-1 protein on the cell surface of various human cancer cells.

### (Example 4) Changes in expression of CAPRIN-1 protein on the cell membrane surface due to suppression of expression of CAPRIN-1 gene>

Among the cancer cell lines in which the expression of the CAPRIN-1 protein on the cell membrane surface was confirmed in Example 3, BT-474, KKU-213, SW780, and OVCAR3 were transfected with each of Stealth RNAi siRNA for CAPRIN-1 (Invitrogen, HSS106231 and HSS106233) and Stealth RNAi siRNA Negative Control (Invitrogen, 12935300) using Lipofectamine RNAiMAX Transfection Reagent (Invitrogen, 13778150) according to a standard method.

Changes in the expression of the CAPRIN-1 protein on the cell membrane surface when the expression of the CAPRIN-1 gene had been suppressed were detected by immunocytochemistry using anti-CAPRIN-1 antibodies according to a standard method. As a result, it was confirmed that suppressing the expression of the CAPRIN-1 gene reduced the expression of the CAPRIN-1 protein on the cell membrane surface of all cancer cells (Figure 1).

### (Example 5) Differences in anchorage-independent colony-forming ability between cancer cell fractions with different expression patterns of the CAPRIN-1 protein on the cell membrane surface>

From cancer cell lines HEC-1-A, SK-MEL-5, and Malme-3M, each of which has two fractions with different expression patterns of the CAPRIN-1 protein on the cell membrane among the cancer cells in which the expression of the CAPRIN-1 protein on the cell membrane surface was confirmed in Example 3, a cancer cell fraction with the low-level expression of the CAPRIN-1 protein on the cell membrane surface (Low; fraction 1) and a cancer cell fraction with the high-level expression of the CAPRIN-1 protein on the cell membrane surface (High; fraction 2) were each separated using a cell sorter (FACS Aria Fusion, BD Biosciences).

The same number of cells of fraction 1 and fraction 2, respectively, were seeded on a 96-well plate for cell culture, and then incubated in a complete medium containing 10% fetal bovine serum under an environment of 37°C and 5% CO₂ for 3 days. Subsequently, CellTiter-Glo Luminescent Cell Viability Assay (Promega, G7575) was used to evaluate cell proliferation, confirming that there was no significant difference in the cell proliferation ability in cell culture using a normal culture flask between fraction 1 and fraction 2. Next, the anchorage-independent colony-forming ability was evaluated by soft agar colony formation assay using the separated cells of fraction 1 and cells of fraction 2. Specifically, 10⁴ cells of each of fraction 1 and fraction 2 were suspended in a DMEM medium gel containing 10% fetal bovine serum containing 0.4% soft agar (Difco, 214220), added to a 35 mm diameter Petri dish, and incubated under an environment of 37°C and 5% CO₂ for 2 to 4 weeks. After incubation, the number of colonies formed in the gel was counted. As a result, the number of colonies formed in fraction 1 was significantly lower than that in fraction 2 in all cancer cells, HEC-1-A, SK-MEL-5, and Malme-3M (Figure 2).

These results demonstrate that the low-level expression of the CAPRIN-1 protein on the membrane surface of the cancer cells reduces the ability of the cancer cells to form anchorage-independent colonies.

### (Example 6) Differences in tumorigenicity in vivo in mice between cancer cell fractions with different expression patterns of CAPRIN-1 protein on the cell membrane surface

The cancer cell fraction with the low-level expression of the CAPRIN-1 protein on the cell membrane surface (Low; fraction 1) and the cancer cell fraction with the high-level expression of the CAPRIN-1 protein on the cell membrane surface (High; fraction 2) separated in Example 5 were each cultured in a normal culture flask using a complete medium containing 10% fetal bovine serum for 4 days to 1 week, and then 1 to 2.5 × 10⁵ cells of fraction 1 and that of fraction 2 were each suspended in 100 µL of DMEM medium containing 25% Matrigel Basement Membrane Matrix (Corning, 354234). Furthermore, the same number of cells were subcutaneously inoculated into NOD-SCID (NOD.CB17-Prkdcscid/NcrCrl) mice under anesthesia. After inoculation of the cancer cells, the size of the cancer of each cancer-bearing mouse was measured over time using a vernier caliper, and the tumor volume was calculated for each individual using the formula: (length of the long axis of the cancer) × (length of the short axis of the cancer)² × 0.5 according to a standard method, and then the average value of the administration group was calculated. As a result, it was found that the tumor growth in the mice inoculated with fraction 2 was significantly faster than the tumor growth in the mice inoculated with fraction 1 (Figure 3).

These results demonstrate that reducing the expression of the CAPRIN-1 protein on the membrane surface of the cancer cells can significantly suppress tumorigenesis *in vivo.*

### (Example 7) Changes in anchorage-independent colony-forming ability of cancer cells through degradation of CAPRIN-1 protein on membrane surface of cancer cells by enzyme treatment

The results of Examples 5 and 6 revealed that reducing the expression of the CAPRIN-1 protein on the membrane surface of the cancer cells significantly reduces the ability to form tumors *in vitro* and *in vivo,* resulting in an anti-tumor effect. Therefore, in this Example, the difference in the anchorage-independent colony-forming ability when reducing the expression of the CAPRIN-1 protein on the cell membrane surface by treating the cancer cells with trypsin, which was a hydrolytic enzyme, was examined through comparison with cancer cells not treated with trypsin.

SK-MEL-5 and OVCAR3, which were the cancer cells confirmed to express the CAPRIN-1 protein on the cell membrane surface in Example 3, were cultured in a normal culture flask using a complete medium containing 10% fetal bovine serum under an environment of 37°C and 5% CO₂. The cultured cancer cell lines SK-MEL-5 and OVCAR3 were collected from the flask using a cell scraper. Portions of the collected cells were treated with a solution containing trypsin, which was a digestive enzyme, (Thermo, Trypsin/EDTA (0.05%)) for 5 minutes, and then the proportions of live cells and dead cells stained with trypan blue were measured and calculated using the trypan blue exclusion method according to a standard method, and then the cell density of live cells was calculated. Through the use of the cancer cells, SK-MEL-5 and OVCAR3, treated with trypsin and SK-MEL-5 and OVCAR3 collected with the cell scraper and not treated with trypsin as described above, the expression of the CAPRIN-1 protein on the cell membrane surface was confirmed by flow cytometry in the same manner as in Example 3. As a result, it was confirmed that the CAPRIN-1 protein was expressed on the cell membrane surface in the above cancer cell lines collected with the cell scraper and not treated. On the other hand, it was confirmed that the expression of the CAPRIN-1 protein on the cell membrane surface was reduced in the cancer cell lines treated with trypsin.

The above-prepared SK-MEL-5 and OVCAR3 expressing the CAPRIN-1 protein on the cell membrane surface, and SK-MEL-5 and OVCAR3 exhibiting the reduced expression of the CAPRIN-1 protein on the cell membrane surface were used to evaluate for the anchorage-independent colony-forming ability by soft agar colony formation assay. 10⁴ cells of each of the above cells were suspended in DMEM medium gel containing 10% fetal bovine serum containing 0.4% soft agar (Difco, 214220), added to a Petri dish with a diameter of 35 mm, and then incubated under an environment of 37°C and 5% CO₂ for 2 to 4 weeks. After incubation, the number of colonies formed in the gel was counted. As a result, the number of colonies formed was significantly lower in both of SK-MEL-5 and OVCAR3 exhibiting the reduced expression of the CAPRIN-1 protein on the cell membrane surface, compared with the above SK-MEL-5 and OVCAR3 expressing the CAPRIN-1 protein on the cell membrane surface.

These results demonstrate that through the reduction of the expression of the CAPRIN-1 protein on the membrane surface of the cancer cells using an enzyme to degrade the CAPRIN-1 protein on the membrane surface of the cancer cells, the ability of the cancer cells to form the anchorage-independent colonies is reduced.

### (Example 8) Changes in anchorage-independent colony-forming ability of cancer cells by treatment with anti-CAPRIN-1 expression-inhibiting antibody

In this example, cancer cells were treated with a rabbit-derived anti-CAPRIN-1 polyclonal antibody, which was one of the CAPRIN-1 expression-inhibiting antibodies, so as to reduce the expression of the CAPRIN-1 protein on the membrane surface of the cancer cells, and thus the anchorage-independent colony-forming ability was examined.

The rabbit-derived anti-CAPRIN-1 polyclonal antibody used herein was prepared by purification using a protein A column from the peripheral blood of rabbits immunized with a recombinant human CAPRIN-1 protein according to a standard method, followed by replacement of the solvent by Dulbecco's PBS (-) with reference to WO2010/016526, WO2011/096517, WO2011/096528, WO2011/096519, WO2011/096533, WO2011/096534, WO2011/096535, WO2013/018886, WO2013/018894, WO2013/018892, WO2013/018891, WO2013/018889, WO2013/018883, WO2013/125636, WO2013/125654, WO2013/125630, WO2013/125640, WO2013/147169, WO2013/147176 and WO2015/020212. For the negative control, peripheral blood of a non-immunized rabbit was purified using a protein A column in the same manner as above, followed by replacement of the solvent by PBS (-) (negative control antibody).

SK-MEL-5, which were the cancer cells confirmed to express the CAPRIN-1 protein on the cell membrane surface in Example 3, was cultured in a normal culture flask using a complete medium containing 10% fetal bovine serum under an environment of 37°C and 5% CO₂. The cultured SK-MEL-5 was collected from the flask using a cell scraper. A portion of the collected cells was reacted with a solution containing the rabbit-derived anti-CAPRIN-1 polyclonal antibody prepared at 0.1 mg/mL at 4°C for 1 hour. On the other hand, as a negative control, SK-MEL-5, which was prepared in the same manner as above and thus reacted with the above negative control antibody, was used to evaluate the anchorage-independent colony-forming ability by soft agar colony formation assay. 10⁴ cells of each of the above cells were suspended in a DMEM medium gel containing 10% fetal bovine serum containing 0.4% soft agar (Difco, 214220), added to a Petri dish with a diameter of 35 mm, and then incubated under an environment of 37°C and 5% CO₂ for 2 to 4 weeks. As a result of counting the number of colonies formed in the gel after incubation, the number of colonies formed was significantly lower in SK-MEL-5 treated with the rabbit-derived anti-CAPRIN-1 polyclonal antibody compared with SK-MEL-5 treated with the negative control antibody.

These results demonstrate that reducing the CAPRIN-1 protein on the membrane surface of the cancer cells with the use of the anti-CAPRIN-1 antibody reduces the ability of the cancer cells to form the anchorage-independent colonies.

### (Example 9) Reduction of expression of CAPRIN-1 protein on membrane surface of cancer cells by low molecular weight compounds>

Dorsomorphin, which was an AMPK inhibitor, (final concentration: 0.1 mM), Wnt agonist 1, which was a Wnt agonist, (final concentration: 20 µM), LY294002, which was an Akt/PI3K inhibitor, (final concentration: 10 µM), Pifithrin-α, which was a P53 inhibitor, (final concentration: 30 µM), Rac1 inhibitor (CAS#1177865-17-6), which was a Rac1 inhibitor, (final concentration: 250 nM), Pyocyanin, which was an ROS generating factor, (final concentration: 20 µM), Mubritinib, which was an ErbB2/HER2 inhibitor, (final concentration: 1 µM), GSK-3β inhibitor VIII, which was a GSK3β inhibitor, (final concentration 10 µM), Dynasore, which was an endocytosis inhibitor, (final concentration 100 µM), and Wnt-C59, which was a Wnt/β-catenin inhibitor, (final concentration: 5 µM), were each added into the wells of a normal culture 6-well plate containing complete medium containing 10% fetal bovine serum, and HCT-116, which were the cancer cells confirmed to express the CAPRIN-1 protein on the cell membrane surface, were cultured under an environment of 37°C and 5% CO₂ for 1 to 3 days. After the culture, HCT-116 treated with various low molecular weight compounds and HCT-116 as a negative control, which were cultured in the same manner with the addition of a solution containing the same amount of DMSO, which was a solvent for various low molecular weight compounds, were collected with a cell scraper, and the expression of the CAPRIN-1 protein on the membrane surface of the cancer cells was confirmed for each by the flow cytometry described in Example 3. As a result, it was confirmed that in all of the groups to which various low molecular weight compounds had been added, the expression of the CAPRIN-1 protein on the membrane surface of the cancer cells was clearly reduced compared to the negative control.

These results revealed that all of the above low molecular weight compounds are substances that reduce the expression of the CAPRIN-1 protein on the membrane surface of the cancer cells.

### (Example 10) Reduction of expression of CAPRIN-1 protein on membrane surface of cancer cells and antitumor effect by AMPK Inhibitor>

The antitumor effect of Dorsomorphin, which was an AMPK inhibitor, confirmed in Example 9 to have the effect of reducing the expression of the CAPRIN-1 protein on the membrane surface of the cancer cells, was confirmed. HCT-116, which were the cancer cells confirmed to express the CAPRIN-1 protein on the cell membrane surface were cultured in a normal culture flask using a complete medium containing 10% fetal bovine serum according to a standard method, and then mixed with RPMI-1640 medium containing 20% Matrigel Basement Membrane Matrix (Corning, 354234), and 1 to 2 × 10⁷ cancer cells per mouse were subcutaneously inoculated into nude mice under anesthesia. After the cancer cells were inoculated, the size of the cancer in each cancer-bearing mouse was measured over time using a vernier caliper, and the tumor volume was calculated for each individual mouse using the formula: (length of the long axis of the cancer) × (length of the short axis of the cancer) 2 × 0.5 according to a standard method, and the average value of the administration group was calculated. After the cancer cells were inoculated, Dorsomorphin was administered at 10 mg/kg twice a week into the tail vein of each cancer-bearing mouse whose tumor size reached 100 to 200 mm³ or more. As a result of the evaluation, an antitumor effect was obtained in which the tumor size of the cancer-bearing mice to which Dorsomorphin had been administered was 70% or less, when the tumor size of the untreated cancer-bearing mice was taken as 100%.

## Claims

1. A pharmaceutical composition for treating and/or preventing a cancer, comprising as an active ingredient a substance that reduces an expression of a CAPRIN-1 protein on a membrane surface of a cancer cell.

2. The pharmaceutical composition according to claim 1, wherein a cancer patient having the cancer is a cancer patient affected with the cancer in which the CAPRIN-1 protein is expressed on the membrane surface of the cancer cell.

3. The pharmaceutical composition according to claim 1 or 2, wherein the substance is a substance that reduces an expression level of the CAPRIN-1 protein on the membrane surface of the cancer cell.

4. The pharmaceutical composition according to any one of claims 1 to 3, wherein the substance is at least one substance selected from the group consisting of an AMPK inhibitor, a Wnt agonist, an Akt/PI3K inhibitor, a P53 inhibitor, a Rac1 inhibitor, a ROS generating factor, an ErbB2/HER2 inhibitor, a GSK3β inhibitor, an endocytosis inhibitor and a Wnt/β-catenin inhibitor.

5. The pharmaceutical composition according to any one of claims 1 to 3, wherein the substance is a substance that suppresses an expression of a CAPRIN-1 gene.

6. The pharmaceutical composition according to claim 5, wherein the substance is a nucleic acid molecule.

7. The pharmaceutical composition according to claim 6, wherein the nucleic acid molecule is at least one nucleic acid molecule selected from the group consisting of a small interfering RNA (siRNA), a micro RNA, a small hairpin RNA (shRNA), a guide RNA (gRNA), an antisense nucleic acid, a ribozyme and a nucleic acid aptamer, targeting the CAPRIN-1 gene.

8. The pharmaceutical composition according to any one of claims 1 to 3, wherein the substance is an enzyme that degrades the CAPRIN-1 protein on the membrane surface of the cancer cell.

9. The pharmaceutical composition according to claim 8, wherein the enzyme is trypsin or chymotrypsin.

10. The pharmaceutical composition according to claim 1 or 2, wherein the substance is a substance that binds to the CAPRIN-1 protein on the membrane surface of the cancer cell.

11. The pharmaceutical composition according to claim 10, wherein the substance is an antibody that binds to the CAPRIN-1 protein on the membrane surface of the cancer cell or an antigen-binding fragment thereof.

12. The pharmaceutical composition according to any one of claims 1 to 11, wherein the cancer is ovarian cancer, bile duct cancer, breast cancer, kidney cancer, pancreatic cancer, colon cancer, melanoma, lung cancer, renal cell carcinoma, head and neck cancer, gastric cancer, biliary tract cancer, brain tumor, prostate cancer, mesothelioma, colorectal cancer, esophageal cancer, gastroesophageal junction cancer, hepatocellular carcinoma, glioblastoma, urothelial carcinoma, bladder cancer, uterine cancer, liver cancer, sarcoma, fibrosarcoma, mast cell tumor, adrenocortical carcinoma, Ewing's tumor, multiple myeloma, testicular cancer, thyroid cancer, basal cell carcinoma, Paget's disease, skin cancer, gastrointestinal stromal tumor (GIST), renal pelvis and ureter cancer, rare cancer, primary central nervous system lymphoma, primary testicular lymphoma, Hodgkin's lymphoma, leukemia, or lymphoma.

13. A method for treating and/or preventing a cancer, comprising administering the pharmaceutical composition according to any one of claims 1 to 12 to a cancer patient.

14. The method for treating and/or preventing the cancer according to claim 13, wherein the cancer patient is a cancer patient affected with a cancer in which a CAPRIN-1 protein is expressed on a membrane surface of a cancer cell.
